# EUROPEAN PATENT APPLICATION

(11) **EP 4 756 685 A1**
(43) Date of publication of application: **10.06.2026**
(21) Application number: 23947001.6
(22) Date of filing: 31.07.2023
(51) Int. Cl.: G06Q 10/06, G16H 10/60

(54) **ACCOUNT ASSOCIATION METHOD FOR DIABETES MANAGEMENT SYSTEM**

(71) Applicant: Medtrum Technologies Inc., Shanghai 201203 (CN)
(72) Inventor: YANG, Cuijun, Shanghai 201203 (CN)
(74) Representative: Vogelbruch, Keang
(86) International application number: PCT/CN2023/110198
(87) International publication number: WO 2025/025046

(57) **Abstract**

The invention discloses an account association method for a diabetes management system, searching within the other account to determine whether the patient account exists in a list of patients of the other accounts; if the patient account does not exist in the list of patients of the other account, sending an account association request to the patient account; the patient account can be associated with the other account by the patient account receives and confirms the account association. For patients, confirming is enough to establish an association, and for others, there is no need to jump between different platforms, so other accounts can be associated with patient accounts through a simple way of operation.

## Description

### TECHNICAL FIELD

The present invention mainly relates to the field of medical device, and in particular to an account association method for a diabetes management system.

### BACKGROUND

The pancreas of healthy people can automatically secrete the required insulin/glucagon according to the glucose level in the human blood, thereby maintaining a reasonable range of blood glucose fluctuations. However, for diabetic patients, the function of their pancreas has been severely compromised, and the pancreas cannot secrete the required dosage of insulin. Therefore, diabetes mellitus is defined as a metabolic disease caused by abnormal pancreatic function, and it is also classified as one of the top three chronic conditions by the WHO. The present medical advancement has not been able to find a cure for diabetes mellitus. Yet, the best the technology could do is control the onset symptoms and complications by stabilizing the blood glucose level for diabetes patients.

Diabetic patients on an insulin pump need to check their blood glucose before infusing insulin into their bodies. At present, most detection methods of continuously monitoring blood glucose are based on in vivo glucose monitoring devices, which use disposable transdermal sensors inserted into the skin to measure glucose concentrations in interstitial fluids and send the blood glucose data through a transmitter to the remote device in real-time for the user to view. This monitoring method is called Continuous Glucose Monitoring (CGM).

Diabetic patients with mild symptoms can complete self-monitoring and management of blood glucose through continuous glucose monitoring, while diabetic patients with more severe symptoms need to be treated by the doctor, such as adopting targeted treatment plans to restore the blood glucose level of diabetic patients to a relatively stable state, and at the same time, providing personalized medication delivery, diet and exercise plans to facilitate the patients to achieve self-monitoring and management of blood glucose. When a doctor treats a diabetic patient, he or she needs to link the doctor's account with the patient's account before viewing the patient's blood glucose data, previous treatment records, etc., and then further adopt a targeted treatment plan. When the patient is an elderly person or a young child, or a person who has a special disease that prevents him/her from self-monitoring and managing his/her blood glucose through continuous glucose monitoring, a guardian is required to monitor and manage the patient's blood glucose, and the guardian needs to associate the patient's account before he/she can view the patient's blood glucose data.

The traditional way of associating a healthcare provider's account or guardian's account with a patient account is that the patient initiates a request on the personal diabetes management device, i.e., enters the follower's (healthcare provider's or guardian's) email address on the personal diabetes management device and sends an email to the healthcare provider or guardian, to request the healthcare provider or guardian to associate his/her account. The process is more complex for the patient, and for healthcare providers or guardians, they need to log in to an email address, i.e., need to jump between different platforms in order to realize the association between the patient's account and the healthcare provider account or guardian account.

Therefore, in the prior art, there is an urgent need for a method that enables the association of a healthcare provider account or a guardian account with a patient account in an easy-to-use manner.

### BRIEF SUMMARY OF THE INVENTION

The embodiment of the present invention discloses an account association method for a diabetes management system, searching within the other account to determine whether the patient account exists in a list of patients of the other accounts; if the patient account does not exist in the list of patients of the other account, sending an account association request to the patient account; the patient account can be associated with the other account by the patient account receives and confirms the account association. For patients, confirming is enough to establish an association, and for others, there is no need to jump between different platforms, so other accounts can be associated with patient accounts through a simple way of operation.

The invention discloses an account association method for a diabetes management system, comprising: providing at least one patient account, at least being configured to view a real-time blood glucose information of the patient; providing at least one other account, searching within the at least one other account to determine whether the patient account exists in a list of patients of the other accounts; if the patient account does not exist in the list of patients of the other account, sending an account association request to the patient account; the patient account, upon receiving the account association request, confirms the account association request to establish an association between the patient account and the at least one other account.

According to one aspect of the present invention, the at least one other account is healthcare provider account and/or guardian account.

According to one aspect of the present invention, the healthcare provider account consists of an HCP main account and/or an HCP sub-account.

According to one aspect of the present invention, the list of patients in the HCP main account and the list of patients the HCP sub-account is consistent after the healthcare provider account is associated with the patient account.

According to one aspect of the present invention, a carrier of the patient account is a personal diabetes management device.

According to one aspect of the present invention, the personal diabetes management device includes at least one of a specialized handheld device, a smartphone and a personal computer.

According to one aspect of the present invention, when a plurality of the personal diabetes management devices is used by the patient at the same time, the plurality of personal diabetes management devices receives the account association request at the same time.

According to one aspect of the present invention, when the account association request is confirmed by one of the plurality of personal diabetes management devices, the other plurality of personal diabetes management devices no longer display the account association request.

According to one aspect of the present invention, a carrier of the other account is a secondary management device, the secondary management device limits the patient's operations on the personal diabetes management device by means of a locking mode.

According to one aspect of the present invention, the secondary management device is a carrier for a guardian account or a healthcare provider account and includes at least one of a specialized handheld device, a smartphone, and a personal computer.

According to one aspect of the present invention, when a plurality of the secondary management devices is used by the healthcare provider or the guardian at the same time, any one of the plurality of secondary management devices is able to send an account association request to the patient account

According to one aspect of the present invention, upon confirmation of the account association request by the patient account, any one of the plurality of secondary management devices is able to view the patient's blood glucose information.

According to one aspect of the present invention, if it is clear that the patient account is not associated prior to searching for the patient account, then the account association request is sent directly to the patient account.

According to one aspect of the present invention, after the patient account and the other account are associated, both the patient account and the other account association are able to disassociate actively.

The invention also discloses account association method for a diabetes management system, comprising: providing at least one patient account, at least being configured to view a real-time blood glucose information of the patient; providing at least one other account, searching within the at least one other account to determine whether the patient account exists in a list of patients of the other accounts; if the patient account does not exist in the list of patients of the other account, a patient exclusive code is generated based on a personal information of the patient and an identifier information of a blood glucose monitoring device used by the patient; the patient account identifies the exclusive code to associate the patient account with the other account.

According to one aspect of the present invention, the identifier is set in the form of a QR code, barcode, or NFC tag.

Compared with the prior art, the technical solution of the present invention has the following advantages:
In the account association method for a diabetes management system disclosed in the present invention, searching the patient account within the other account to confirm whether the patient account exists in a list of patients of the other accounts; if the patient account does not exist in the list of patients of the other account, sending an account association request to the patient account; the patient account can be associated with the other account by the patient account receives and confirms the account association. For patients, confirming is enough to associate, and for others, there is no need to jump between different platforms, so other accounts can be associated with patient accounts through a simple way of operation.

Furthermore, the healthcare provider account includes the HCP main account and the HCP sub-account. After the healthcare provider account is associated with the patient account through the HCP main account or the HCP sub-account, the list of patients in the HCP main account and the HCP sub-account is the same, and information can be shared among different healthcare provider, which further simplifies the operation of the healthcare provider.

Furthermore, the carrier of the patient account is a personal diabetes management device, and when the patient uses a plurality of personal diabetes management devices at the same time, the plurality of personal diabetes management devices will receive the account association request at the same time, and when the patient confirms the account association request through one of the plurality of personal diabetes management devices, the other plurality of personal diabetes management devices will no longer display the account association request, which facilitates the patient to monitor and manage the blood glucose level using different carriers in different situations, and achieves the real-time monitoring of the patient's blood glucose level, but at the same time, will not increase the difficulty of operation.

Furthermore, if it is clear that the patient account is not associated prior to searching for the patient account, sending the account association request directly to the patient account may further simplify the account association method.

In the account association method for a diabetes management system disclosed in the present invention, searching the patient account within the other account to confirm whether the patient account exists in a list of patients of the other accounts; if the patient account does not exist in the list of patients of the other account, a patient exclusive code is generated based on a personal information of the patient and an identifier information of a blood glucose monitoring device used by the patient; the patient account identifies the exclusive code to associate the patient account with the other account. For patients, identifying the patient exclusive code is enough to associate, and for others, there is no need to jump between different platforms, so other accounts can be associated with patient accounts through a simple way of operation.

### BRIEF DESCRIPTION OF THE DRAWINGS

FIG.1 is a schematic diagram of the blood glucose monitoring system according to an embodiment of the present invention.
FIG.2 is a schematic diagram of the integrated continuous glucose monitoring device according to an embodiment of the present invention.
FIG.3 is a schematic diagram of the split continuous glucose monitoring device according to an embodiment of the present invention.
FIG.4 is a schematic diagram of the interface of the APP in use when the patient account receives an account association request according to an embodiment of the present invention.
FIG.5 is a schematic diagram of the interface of the APP in idle when the patient account receives an account association request according to an embodiment of the present invention.
FIG.6 is flowchart of a method of associating accounts according to an embodiment of the present invention.

### DETAILED DESCRIPTION

As mentioned above, the current account association method used in the diabetes management system is cumbersome and the process is complicated for patients, and for healthcare providers or guardians, they need to log in to an email address, i.e., need to jump between different platforms in order to realize the association between the patient account and the healthcare provider account or guardian's account.

In order to solve this problem, the present invention provides an account association method for a diabetes management system, searching the patient account within the other account to confirm whether the patient account exists in a list of patients of the other accounts; if the patient account does not exist in the list of patients of the other account, sending an account association request to the patient account; the patient account can be associated with the other account by the patient account receives and confirms the account association. For patients, confirming is enough to associate, and for others, there is no need to jump between different platforms, so other accounts can be associated with patient accounts through a simple way of operation.

Various exemplary embodiments of the present invention will now be described in detail with reference to the drawings. The relative arrangement of the components and the steps, numerical expressions and numerical values set forth in the embodiments are not to be construed as limiting the scope of the invention.

In addition, it should be understood that, for ease of description, the dimensions of the various components shown in the figures are not necessarily drawn in the actual scale relationship, for example, the thickness, width, length or distance of certain units may be exaggerated relative to other parts.

The following description of the exemplary embodiments is merely illustrative, and is not intended to be in any way limiting the invention and its application or use. The techniques, methods, and devices that are known to those of ordinary skill in the art may not be discussed in detail, but such techniques, methods, and devices should be considered as part of the specification.

It should be noted that similar reference numerals and letters indicate similar items in the following figures. Therefore, once an item is defined or illustrated in a drawing, it will not be discussed further in the following description of the drawings.

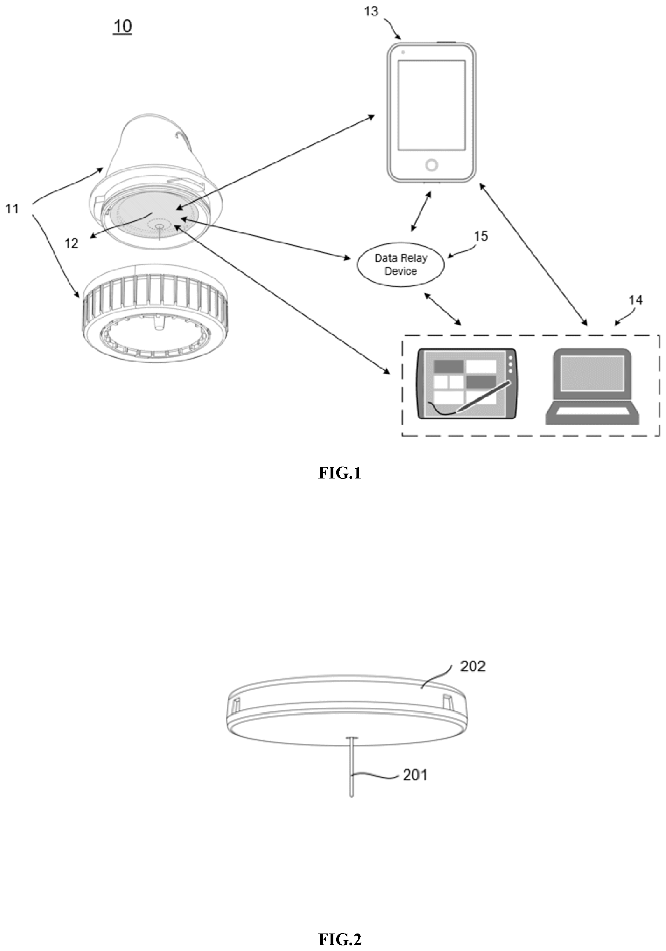

FIG.1 is a schematic diagram of the blood glucose monitoring system.

The blood glucose monitoring system 10 includes an auxiliary applicator 11, a blood glucose monitoring device 12, and a personal diabetes management device (hereinafter referred to as the management device) 13. The auxiliary applicator 11 is used to install the blood glucose monitoring device 12 onto the surface of the skin of a user, and after the blood glucose monitoring device 12 is installed onto the surface of the skin of the user, it obtains information on the concentration of the blood glucose in the body of the user and sends the information on the concentration of the blood glucose to the management device 13. The management device 13 receives the blood glucose concentration information and then analyzes and sort off it, and ultimately displays it in an intuitive manner on a display screen of the management device 13. The management device 13 includes a dedicated handheld device, a smartphone that can download a dedicated APP, a personal computer that can access a specialized website, a tablet PC, and the like.

In other embodiments of the present invention, the blood glucose monitoring system 10 further comprises a diabetes secondary management device (hereinafter referred to as the secondary management device) 14. The secondary management device 14 may be a dedicated handheld device, a smartphone that can download a dedicated APP, a personal computer that can access a specialized website, a tablet PC, etc., and the user thereof may be the patient him/herself or a person other than the patient, such as, for example, the patient's guardian or a doctor.

In some embodiments of the present invention, the blood glucose monitoring device 12 is wirelessly communicatively connected to the management device 13 and/or the secondary management device 14 via Bluetooth, Wi-Fi, or the Internet. In one embodiment of the present invention, the blood glucose monitoring device 12 is wirelessly communicatively connected to the management device 13 and/or the secondary management device 14 via Bluetooth, which can save the power consumption of the blood glucose monitoring device 12 and prolong its service life.

In some embodiments of the present invention, the management device 13 may establish a wireless communication connection with the secondary management device 14 by means of Bluetooth, Wi-Fi, or Internet, or establish a wired communication connection with the secondary management device 14 by means of USB, serial, Ethernet, or the like, in order to realize data transmission between the management device 13 and the secondary management device 14.

In an embodiment of the present invention, the blood glucose monitoring device 12 is a wearable continuous glucose monitor (hereinafter referred to as CGM), the CGM comprising a sensor and a transmitter, the sensor is used to collect the blood glucose level in the body of the human being and transmit the collected blood glucose level information, and the transmitter is connected to the sensor, and used to receive the blood glucose level information transmitted by the sensor which is implanted under the skin, and converting the blood glucose level information into a wireless signal to output. Each CGM has a unique identifier including, but not limited to, a device identifier, a hardware identifier, a generic unique identifier, a serial number, a communication protocol based identifier (e.g., a BLE ID), a manufacturer's identifier, etc., Preferably, the identifier is a serial number of the CGM, which is formed by a plurality of randomly-combined numerical and alphabetic combinations, and which can be set on the CGM's housing or packaging, and which may also be different depending on the different types of CGMs.

Specifically, in one embodiment of the present invention, the CGM is integrated, i.e., the sensor and the transmitter have been integrated together prior to use, and is a single-use product, which is discarded after use, as shown in FIG. 2. FIG.2 is a schematic diagram of the integrated continuous glucose monitoring device according to an embodiment of the present invention. The integrated continuous glucose monitoring device includes a sensor 201, a housing 202, and a transmitter (not shown in the figure) set inside the housing 202, the sensor 301 is used to monitor the patient's body fluid blood glucose data information, and transmit the said blood glucose data information to the transmitter through an internal circuit, which in turn sends it to a receiver. The identifier may be provided on the outer housing of the CGM or on the outer packaging or within the CGM.

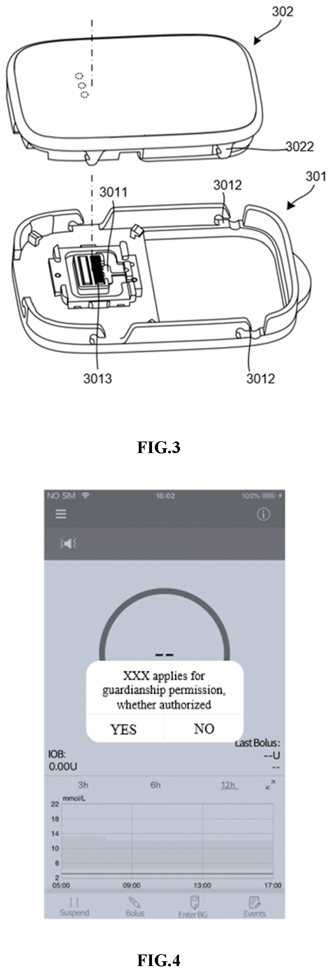

In another embodiment of the present invention, the CGM is split, i.e., the sensor and the transmitter are two different parts prior to use, packaged separately, and only integrated together when in use, as shown in FIG. 3. FIG.3 is a schematic diagram of the split continuous glucose monitoring device according to an embodiment of the present invention. The split continuous glucose monitoring device includes a base 301 and a transmitter 302, the base is provided with a sensor 3011, the transmitter 302 has a separate housing, the housings of the base 301 and the transmitter 302 are respectively provided with snap-in structures 3012 and 3022, the base 301 and the transmitter 302 are snapped into a whole through the snap-in structures when in use, the sensor 3011 is electrically connected to the transmitter 302 through the electrical connection 3013, the sensor 301 is used to monitor the patient's blood glucose data information, the blood glucose data information is transmitted to the transmitter 302 through the electrical connection 3013, and then sent to the receiver by the transmitter 302.

In one embodiment of the present invention, both the sensor and the transmitter of the split continuous glucose monitoring device are single-use products, which are discarded after use, and therefore the identifier may be provided on the housing or the outer packaging of the sensor or the transmitter. In yet another embodiment of the present invention, only the sensor of the split continuous glucose monitoring device is a single-use product and the transmitter is a reusable product, therefore, preferably, in this embodiment, the identifier is provided on the housing or the outer packaging of the transmitter, which reduces the frequency of tying the patient's information to the identifier and improves the patient experience. This will be described in more detail below.

When the identifier is set on the housing or outer packaging of the CGM or transmitter, it is set in a form that includes, but is not limited to, a QR code, a bar code, or an NFC tag.

Before using the continuous glucose monitoring system to monitor the patient's blood glucose information, the patient needs to create a new account on the management device 13 to establish a connection between the continuous glucose monitoring device 12 and the management device 13, which can be realized by manually enter the patient's personal information, and at the same time, manually enters or scans the QR code, the bar code, or the NFC tag on the housing or on the outer packaging of the CGM to pair the management device 13 and the blood glucose monitoring device 12 and activate the blood glucose monitoring device 12, and then the blood glucose monitoring device 12 communicates with the management device 13 via Bluetooth, Wi-Fi, or Internet, and sends the monitored blood glucose data to the management device 13 in real time.

When the CGM is split and the transmitter is reusable, the identifier of the CGM is set on the housing or packaging of the transmitter, and the patient only needs to replace the sensor when replacing the CGM without replacing the transmitter, and the identifier of the CGM remains unchanged, and thus there is no need to update the information of the identifier of the CGM through the management device 13, which reduces the operation of the patient and improves the user experience.

In one embodiment of the present invention, when the user of the secondary management device 14 is a healthcare provider or a guardian, after the healthcare provider or the guardian creates his or her own account on the secondary management device 14, the healthcare provider or the guardian needs to associate the healthcare provider's or the guardian's account with the patient's account in order to view the patient's the blood glucose data, the treatment record, and so on, and further take a targeted treatment plan.

The traditional way of associating a healthcare provider's account or guardian's account with a patient account is that the patient initiates a request on the personal diabetes management device, i.e., enters the follower's (healthcare provider's or guardian's) email address on the personal diabetes management device and sends an email to the healthcare provider or guardian, to request the healthcare provider or guardian to associate his/her account. The process is more complex for the patient, and for healthcare providers or guardians, they need to log in to an email address, i.e., need to jump between different platforms in order to realize the association between the patient's account and the healthcare provider's account or guardian's account.

In the embodiment of the present invention, a relatively simple operation method is provided. Healthcare provider searches for the account name of the patient they want to associate through the secondary management device 14, sends an association request to the patient, and the personal management device 13 used by the patient will receive an association request notification. When the management device 13 is a smartphone, it communicates with the blood glucose monitoring device 12 through a dedicated APP, if the dedicated APP is in use, the notification will display "XXX applies for guardianship permission, whether authorized" in text and/or other forms, as shown in FIG.4. Patient can associate with healthcare provider accounts by clicking " YES ", or not authorize association with healthcare provider accounts by clicking "NO". If the patient accidentally clicks "NO", they can also find the associated request on the remote monitoring page and accept it by clicking "YES".

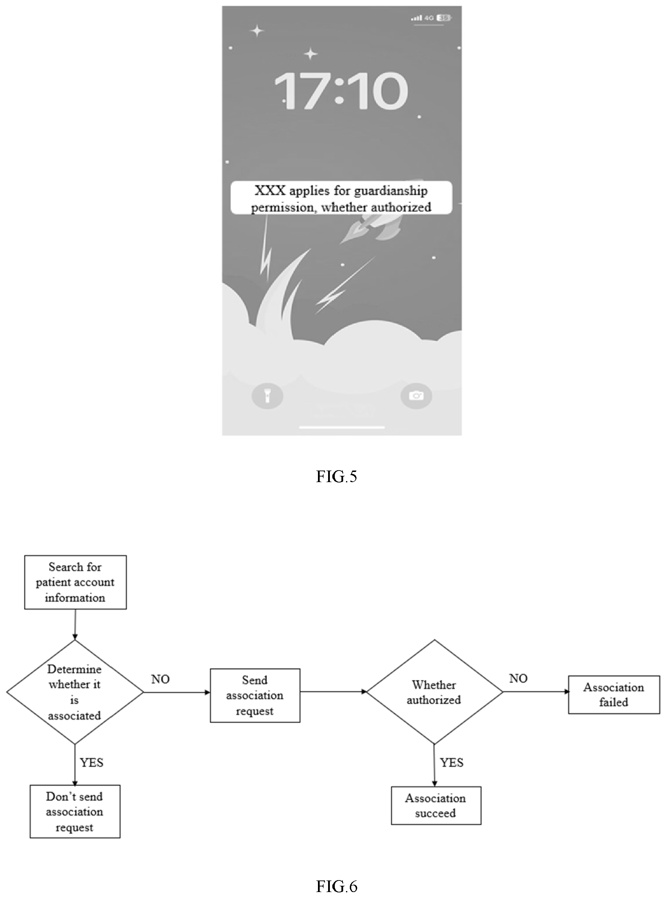

If the APP is in idle, the notification will display "XXX applies for guardianship permission, whether authorized", as shown in FIG.5. The patient clicks on the notification to enter the APP, and the interface will be shown as in FIG.4. The patient can authorize or not authorize the association with the healthcare provider account by clicking "YES" or "NO". If the patient does not respond within a certain period of time, such as one minute or 30 seconds, the backend of the APP will send notifications at a predetermined frequency, such as every five minutes, or as time increases, the interval between notifications will gradually increase, and the notification displays "XXX applies for guardianship authority, whether authorized" until the patient responds.

It should be noted that before the healthcare provider uses the secondary management device 14 to send an association request to the patient account, the account information of the patient to be associated can be searched first to confirm whether the patient account information already exists in the patient list, as shown in FIG.6. FIG.6 is the flowchart of the account association method used in the embodiment of the present invention. If the patient account information already exists in the patient list, it indicates that the patient account has been successfully associated, there is no need to issue another association request , so no association request will be sent. If the patient account information does not exist in the patient list, it indicates that the patient account has not been successfully associated. Then, an association request is issued to the patient. After receiving the association notification, if the patient accepts the association request, it can be successfully associated with the patient account. If the patient does not accept the association request, it indicates that the patient refuses to accept the association. The patient can authorize or does not authorize the association with the health provider account by clicking "YES" or "NO".

When the management device 13 is a dedicated handheld device and the dedicated handheld device is in an unlocked state, a notification as shown in FIG.4 will be displayed on the interface. Patients can associate their accounts with healthcare provider's account by clicking "YES", or not authorize their association with healthcare provider's account by clicking "NO".

When the dedicated handheld device is in the locked state, a notification will be displayed on the locked screen interface, displaying "XXX applies for guardianship permission, whether authorized". After the patient unlocks, click on the notification, and the notification as shown in FIG.4 will be displayed on the interface. The patient can associate with the healthcare provider's account by clicking "YES", or not authorize the association with the healthcare provider's account by clicking "NO". If the patient does not respond within a certain period of time, such as one minute or 30 seconds, the associated request notification will temporarily disappear. When the dedicated handheld device is in the locked state again, the locked interface will display the notification again.

In another embodiment of the present invention, patients can also receive association requests from healthcare provider through a dedicated website. Similarly, when healthcare provider confirm that the patient's account information does not exist in the patient list, an account association request is sent to the patient's account. After accessing the dedicated website and logging into the personal account, the patient requests notification "XXX applies for guardianship permission, whether authorized" to be displayed on the main page. The patient authorizes or does not authorize the association with the healthcare provider account by clicking "YES" or "NO". If the patient does not respond within a certain period of time, such as one minute or 30 seconds, the notification will temporarily disappear and reappear on the main page every time when the patient logs in to their personal account. The notification will be displayed on the main page at a predetermined frequency, such as every five minutes, or as time increases, the interval between notifications will gradually increase, and the notification displayed as "XXX applies for guardianship authority, whether authorized" until the patient responds.

It should be noted that patients can use one or more specialized handheld devices, smartphones, or personal computers to monitor blood glucose level. Therefore, when the patient uses at least two devices simultaneously, after the healthcare provider sends an association request, and all the devices used by patients will receive association request notifications. When the patient responds to association request notifications in one of the devices, the associated request notifications on other usage devices will disappear and will not reappear. If patient uses dedicated handheld devices, smartphones, and personal computers to monitor the real-time blood glucose information at the same time, when healthcare providers send association requests, all the dedicated handheld devices, smartphones, and personal computers will receive association request notifications. If the patient first receives association request notifications on their smartphones and click "YES" in a timely manner to associate with healthcare provider accounts, the notifications on dedicated handheld devices and personal computers will disappear, but they will be retained in the history notification record and marked as processed.

The secondary management device 14 used by healthcare provider can be equipped with one or more processors, allowing multiple individuals with different access levels to modify the setting parameters of the secondary management device 14. Such as the attending physician is configured with the highest level of access to the HCP main account, the HCP main account can create sub accounts, add patients, and send an association request to the patient account. After the patient account authorizes to the association request, all patient information, and the operation records of the main account and sub accounts can be viewed by the HCP main account. While the general doctor or nurse who is configured with access to HCP sub account can add patients, that is, send an association request to the patient account, after the patient account agrees to the association request, all patient information in the department can be viewed by the HCP sub account. Therefore, healthcare provider can send association requests through the HCP main account or HCP sub account. After the healthcare provider account and patient account are successfully associated, the patient account will be displayed in the patient list. The patient list between the HCP main account and the HCP sub account is consistent, making it convenient for doctors and nurses in the same department or section to manage patient accounts, view patient blood glucose information, and also view the operation records and time of the main account and each sub account, such as view records and time of the operation for adding or deleting patient account.

It should be noted that healthcare provider can send patient account association requests to patients through any specialized handheld device, smartphone app, or personal computer. Patients can also receive association requests through any specialized handheld device, smartphone, or personal computer. After the healthcare provider account is associated with the patient's account, any device of the secondary management device of the healthcare provider can view the patient's blood glucose information, making it convenient for healthcare provider to monitor the patient's blood glucose information in different ways according to their needs at different times, achieving real-time monitoring of the patient's blood glucose level.

In one embodiment of the present invention, when the patient account is associated with the healthcare provider account, the content displayed on the management device 13 used by the patient and the secondary management device 14 used by the healthcare provider are consistent.

In another embodiment of the invention, when the patient is an old person or a young child, or a diabetes patient with a special disease who cannot use the management device 13 for self-monitoring the blood glucose level, the healthcare provider can open the locking mode through the secondary management device 14 to limit the operation of the patient on the management device 13, so that the patient cannot view the real-time blood glucose data on the management device 13, cannot receive blood glucose alarms, cannot change the alarm settings, etc, It can prevent patients from mis-operating the management device 13, such as deleting the device, disconnecting the connection between the management device 13 and CGM, thereby affecting the normal blood glucose monitoring of CGM. It also can be set as not accept blood sugar alarms, which can also prevent interference or impact on patients caused by blood sugar alarms.

When the user of the secondary management device 14 is the guardian, the guardian can also monitor the patient's blood sugar level by associating the patient account with the secondary management device 14, such as a dedicated handheld device, smartphone, or personal computer. The association method is the same as the association method between the healthcare provider account and the patient account mentioned above, that is, the guardian searches the patient account through the secondary management device 14 and sends an association request to the patient account, patients can associate guardian accounts with patient accounts by receiving association requests from guardians through secondary management device 14.

Similarly, before the guardian uses the secondary management device 14 to send an association request to the patient account, the account information of the patient to be associated can be searched first to determine whether the patient account information already exists in the patient list, as shown in FIG.6. If the patient account information already exists in the patient list, it indicates that the patient account has been successfully associated, there is no need to issue another association request, so no association request will be sent. If the patient account information does not exist in the patient list, it indicates that the patient account has not been successfully associated. Then, an association request is issued to the patient. After receiving the association notification, if the patient accepts the association request, it can be successfully associated with the patient account. If the patient does not accept the association request, it indicates that the patient refuses to accept the association. The patient can authorize or does not authorize the association with the guardian account by clicking "YES" or "NO".

It should be noted that guardians can send patient account association requests to patients through any specialized handheld device, smartphone, or personal computer, and patients can use one or more specialized handheld devices, smartphones, or personal computers to accept the association request and monitor blood glucose level. Therefore, when the patient uses at least two devices simultaneously, after the guardian sends an association request, and all the devices used by patients will receive association request notifications. When the patient responds to association request notifications in one of the devices, the associated request notifications on other usage devices will disappear and will not reappear. After the guardian account is associated with the patient's account, any one of the guardian's secondary management devices can view the patient's blood glucose information, making it convenient for the guardian to monitor the patient's blood glucose information in different ways according to their needs at different times, achieving real-time monitoring of the patient's blood glucose levels.

When the patient is an old person or a young child, or a diabetes patient with a special disease who cannot use the management device 13 for self-monitoring the blood glucose level, the guardian can open the locking mode through the secondary management device 14 to limit the operation of the patient on the management device 13, so that the patient cannot view the real-time blood glucose data on the management device 13, cannot receive blood glucose alarms, cannot change the alarm settings, etc, It can prevent patients from mis-operating the management device 13, such as deleting the device, disconnecting the connection between the management device 13 and CGM, thereby affecting the normal blood glucose monitoring of CGM. It also can be set as not accept blood sugar alarms, which can also prevent interference or impact on patients caused by blood sugar alarms.

A patient account can be associated with multiple guardian accounts and healthcare provider accounts. A guardian account or a healthcare provider account can be associated with multiple patient accounts. After the association between the healthcare provider account or guardian account and the patient account is completed, all healthcare provider accounts and guardian accounts will be displayed in the remote monitoring list of the patient's personal management device. Similarly, the information of all patient accounts that healthcare provider or guardians are interested in will also be displayed in their respective patient lists. When the association is no longer needed, each party can actively terminate the association. The guardian or healthcare provider only need to delete the patient from the patient list, or the patient only needs to delete the guardian or healthcare provider account from the remote monitoring list.

In one embodiment of the invention, after the healthcare provider or guardian searches the account information of the patient they want to associate and confirms that the patient's account information does not exist in the patient list, they can directly fill in the patient's personal information, such as name, gender, age, hospital, department, clinical diagnosis, ID number, inpatient number/outpatient number, CGM identifier and other information, and then generate the exclusive code of the patient, such as QR code, bar code, verification code, etc.. The information should be filled include at least the name and CGM identifier information. CGM identifier information, as mentioned earlier, includes but is not limited to device identifier, hardware identifier, universal unique identifier, serial number, communication protocol based identifier (such as BLE ID), manufacturer's identifier, etc. Preferably, identifier information is the CGM serial number, formed by a combination of multiple randomly combined numbers and letters. It can be entered manually or by scanning QR codes, barcodes, or NFC labels on the CGM housing or outer packaging. Patients can identify their exclusive codes through the management device 13, such as scanning exclusive QR codes, barcodes, or manually entering verification codes, barcodes, etc., to achieve association with healthcare provider accounts or guardian accounts and patient accounts.

In another embodiment of the invention, after the healthcare provider searches the account information of the patient they want to associate, and confirm that the patient's account information does not exist in the patient list, they can directly fill in the patient's personal information, such as name, gender, age, hospital, department, clinical diagnosis, ID number, inpatient number/outpatient number, CGM identifier, patient's mobile number and the verification code obtained through the patient's mobile number, and then click YES, the patient name will be directly displayed in the patient list. That is, at that time, the healthcare provider can view the patient's blood glucose information through the secondary management device 14, while the patient does not need to use the management device 13, meaning that the patient does not need to pay attention to or view their own blood glucose information, and it is entirely up to healthcare provider to monitor the patient's blood glucose information in real time and take corresponding treatment plans. This is particularly suitable for patients who are elderly or young children, or those with special illnesses. If the patient still wants to monitor or view blood glucose information on their own, they can use the management device 13 to create a new account and establish a connection between the blood glucose monitoring device 12 and the management device 13. After establishing the connection, the management device 13 used by the patient and the secondary management device 14 used by healthcare provider can simultaneously view the patient's blood glucose information, but healthcare provider cannot lock the management device 13 used by the patient through the secondary management device 14, thus unable to restrict the patient's operations on the management device 13.

In summary, the present invention discloses an account association method for a diabetes management system, searching the patient account within the other account to confirm whether the patient account exists in a list of patients of the other accounts; if the patient account does not exist in the list of patients of the other account, sending an account association request to the patient account; the patient account can be associated with the other account by the patient account receives and confirms the account association. For patients, confirming is enough to associate, and for others, there is no need to jump between different platforms, so other accounts can be associated with patient accounts through a simple way of operation.

While the invention has been described in detail with reference to the specific embodiments of the present invention, it should be understood that it will be appreciated by those skilled in the art that the above embodiments may be modified without departing from the scope and spirit of the invention. The scope of the invention is defined by the appended claims.

## Claims

1. An account association method for a diabetes management system, comprising:
providing at least one patient account, at least being configured to view a real-time blood glucose information of the patient;
providing at least one other account, searching within the at least one other account to determine whether the patient account exists in a list of patients of the at least one other accounts; wherein
if the patient account does not exist in the list of patients of the other account, sending an account association request to the patient account;
the patient account, upon receiving the account association request, confirms the account association request to establish an association between the patient account and the at least one other account..

2. The account association method for a diabetes management system of claim 1, wherein, the at least one other account is healthcare provider account and/or guardian account.

3. The account association method for a diabetes management system of claim 2, wherein,
the healthcare provider account consists of an HCP main account and/or an HCP sub-account.

4. The account association method for a diabetes management system of claim 3, wherein,
the list of patients in the HCP main account and the list of patients the HCP sub-account is consistent after the healthcare provider account is associated with the patient account.

5. The account association method for a diabetes management system of claim 1, wherein, a carrier of the patient account is a personal diabetes management device.

6. The account association method for a diabetes management system of claim 5, wherein,
the personal diabetes management device includes at least one of a specialized handheld device, a smartphone and a personal computer.

7. The account association method for a diabetes management system of claim 6, wherein,
when a plurality of the personal diabetes management devices is used by the patient at the same time, the plurality of personal diabetes management devices receives the account association request at the same time.

8. The account association method for a diabetes management system of claim 7, wherein,
when the account association request is confirmed by one of the plurality of personal diabetes management devices, the other plurality of personal diabetes management devices no longer display the account association request.

9. The account association method for a diabetes management system of claim 8, wherein,
a carrier of the other account is a secondary management device, the secondary management device limits the patient's operations on the personal diabetes management device by means of a locking mode.

10. The account association method for a diabetes management system of claim 9, wherein,
the secondary management device is a carrier for a guardian account or a healthcare provider account and includes at least one of a specialized handheld device, a smartphone, and a personal computer.

11. The account association method for a diabetes management system of claim 10, wherein,
when a plurality of the secondary management devices is used by the healthcare provider or the guardian at the same time, any one of the plurality of secondary management devices is able to send an account association request to the patient account.

12. The account association method for a diabetes management system of claim 11, wherein,
upon confirmation of the account association request by the patient account, any one of the plurality of secondary management devices is able to view the patient's blood glucose information.

13. The account association method for a diabetes management system of claim 1, wherein,
if it is clear that the patient account is not associated prior to searching for the patient account, then the account association request is sent directly to the patient account.

14. The account association method for a diabetes management system of claim 1, wherein,
after the patient account and the other account are associated, both the patient account and the other account association are able to disassociate actively.

15. An account association method for a diabetes management system, comprising:
providing at least one patient account, at least being configured to view a real-time blood glucose information of the patient;
providing at least one other account, searching within the at least one other account to determine whether the patient account exists in a list of patients of the other accounts;
wherein
if the patient account does not exist in the list of patients of the other account, a patient exclusive code is generated based on a personal information of the patient and an identifier information of a blood glucose monitoring device used by the patient;
the patient account identifies the exclusive code to associate the patient account with the other account.

16. The account association method for a diabetes management system of claim 15, wherein, the identifier is set in the form of a QR code, barcode, or NFC tag.
